# EUROPEAN PATENT SPECIFICATION

(11) **EP 1 976 489 B1**
(45) Date of publication and mention of the grant of the patent: **18.07.2012**
(21) Application number: 07708601.5
(22) Date of filing: 25.01.2007
(51) Int. Cl.: A61K 9/20, A61K 31/38, A61K 9/22, A61K 9/16, A61K 9/50

(54) **MULTIPLE UNIT TYPE SUSTAINED RELEASE ORAL FORMULATION COMPRISING ZALTOPROFEN AND PROCESS FOR THE PREPARATION THEREOF**
AUS MEHREREN EINHEITEN BESTEHENDE ORALE FORMULIERUNG MIT VERZÖGERTER FREISETZUNG ENTHALTEND ZALTOPROFEN UND HERSTELLUNGSVERFAHREN DAFÜR
FORMULATION POUR VOIE ORALE À LIBÉRATION PROLONGÉE ET À PLUSIEURS UNITÉS COMPRENANT ZALTOPROFEN ET PROCÉDÉ DE PRÉPARATION DE LADITE FORMULATION

(30) Priority: 27.01.2006 KR 20060009057
(43) Date of publication of application: 08.10.2008
(73) Proprietor: CJ CheilJedang Corporation, Jung-gu Seoul 100-802 (KR)
(72) Inventor: NOH, Hyun Jung, Gyeonggi-do 463-792 (KR); CHO, Cheong Weon, Gyeonggi-do 463-776 (KR); KU, Jeong, Gyeonggi-do 449-574 (KR); KIM, Taek Rho, Seoul 137-852 (KR); KANG, Hee Chol, Daejeon 301-783 (KR); CAO, Qing Ri, Gyeonggi-do 443-800 (KR); YANG, Eun Young, Gyeonggi-do 449-914 (KR); AN, Tae Kun, Gyeonggi-do 449-780 (KR); JEON, Eun Kyung, Gyeonggi-do 449-718 (KR); KO, Jae Kyoung, Gyeonggi-do 446-773 (KR); HONG, Hye Suk, Gyeonggi-do 463-820 (KR); KIM, Il Hwan, Daejeon 306-777 (KR); SUH, Hea Ran, Seoul 100-802 (KR); HAN, Hye Jin, Gyeonggi-do 441-750 (KR); CHAE, Gang Soo, Jeollabuk-do 573-110 (KR)
(74) Representative: Mai, Dörr, Besier
(86) International application number: PCT/KR2007/000439
(87) International publication number: WO 2007/086692

(56) References cited:
- EP-A1- 0 305 051
- WO-A1-2006/015485
- WO-A2-2004/024128
- US-A- 4 002 731
- US-A- 4 720 387
- US-A- 4 720 387
- US-A- 5 093 200
- US-A1- 4 892 741
- US-A1- 2005 106 248

## Description

### Technical Field

The present invention relates to a sustained release oral formulation, and more particularly, to a multiple unit type sustained release oral formulation comprising both a sustained release part and a rapid release part.

### Background Art

The advantages of sustained release formulations, which are widely known in the field of pharmaceutics, are well known in general. Among such advantages, included are that the concentration of a drug in the blood can be maintained at a desirable level for a relatively long time so that the frequency of dosage required to achieve the same effect as the conventional dosage may be decreased, and also that as a result, the patient's compliance to the drug can be enhanced. Furthermore, while a particular pharmacological treatment requires administration of a drug in a sequential manner, thereby excellent treating effects being possibly expected, such pharmacological treatment can be practiced by a patient by complying with the prescription regimen following a regulated time schedule, but there are many cases where it is often difficult to obtain the expected therapeutic effects because of the non-compliance of the patients. Therefore, development of sustained release formulations which can continuously release a drug, which otherwise should be repeatedly administered several times a day so as to maintain an effective plasma concentration of the drug, may contribute to simplification of the treatment, and to reduction or elimination of the risk of inappropriate administration. The need for such formulations is currently urgent in the case of non-steroidal anti-inflammatory drugs which are presently subject to undesirable chronic administration.

In order to minimize the effect of missed doses of a drug caused by insufficient compliance of a patient, and to maintain the therapeutic blood concentration of the drug, a number of techniques to provide release controllability and extended release formulations have been used. Drugs that are administered in conventional simple tablet or capsule formulations tend to exhibit a rate of drug reaching the body fluid, which is very high initially and then drastically drops afterwards. Such pattern results in many drugs exhibiting a temporary excessive blood concentration of drug, and a subsequent drug concentration that is therapeutically insufficient. Such problems have limited the clinical use of the formulations. This delivery pattern was improved by introduction of various controlled delivery systems in the 1970's. These systems providing relatively constantly controlled drug delivery enabled excessive blood concentration and insufficient maintenance of blood concentration of a drug to be avoided. This technology has led to provision of effective medicaments with reduced side effects, and a decrease in the dosage frequency.

More specifically, WO 98/01117 discloses a sustained release formulation comprising a sustained release carrier which can release non-steroidal anti-inflammatory drugs over a desired sustained release period (12 to 24 hours), and sustained release excipients that are adequate for the manufacture of such sustained release formulation.

Such sustained release formulations for controlling the dissolution rate of an active ingredient can be classified to single unit type formulations and multiple unit type formulations. A multiple unit type formulation is a formulation having two or more units which are co-present in a single formulation and differ from each other in the drug release rates, as opposed to a single unit type formulation having a single unit. The multiple unit type formulation can be said to be a sustained release formulation which has been further developed from the single unit type formulation, since the multiple unit type formulation has excellent features such as less fluctuation in the absorption of active ingredient, good reproducibility of drug dissolution, and applicability to two or more active ingredients, as compared with the single unit type formulation.

Particularly in the case of non-steroidal anti-inflammatory drugs, since the nature of these drugs requires exhibiting rapid manifestation of efficacy, the drugs need to manifest the efficacy immediately after administration, and to enable the maintenance of the effect for 24 hours. Thus, multiple unit type sustained release formulations which can simultaneously exhibit rapid releasability and sustained releasability, are in need.

Zaltoprofen, a non-steroidal anti-inflammatory drug, has excellent effects against post-surgery or post-trauma chronic inflammation. Zaltoprofen typically requires a dosage of three times a day at a dose of about 80 mg for adults. Therefore, in order to improve patient's convenience and dosage compliance, and to reduce gastrointestinal side effects, it is desirable to have a formulation of once-daily administration, which enables one administration a day. However, there is no report to date on sustained release formulations specifically developed for zaltoprofen.

### Disclosure of Invention

### Technical Problem

Accordingly, it is an object of the present invention to provide a multiple unit type sustained release formulation which facilitates the control of release of zaltoprofen.

It is another object of the invention to provide a method for preparing a multiple unit type sustained release formulation which facilitates the control of release of zaltoprofen.

### Technical Solution

In one aspect to achieve the above-described objects, the present invention provides a multiple unit type controlled release oral formulation comprising:
sustained release pellets formed from granules containing zaltoprofen and a water-insoluble polymer, the granules being coated with a sustained release base material; and
rapid release granules containing zaltoprofen. This sustained release oral formulation may be in the form of a tablet or a capsule.

The granules constituting the sustained release pellets may comprise 5 to 30 parts by weight of the water-insoluble polymer, relative to 100 parts by weight of zaltoprofen in a sustained release pellet. Furthermore, the granules may be coated with 5 to 40 parts by weight of the sustained release base material relative to 100 parts by weight of zaltoprofen in a sustained release pellet, to form the sustained release pellets.

The ratio of zaltoprofen contained in the sustained release pellets to zaltoprofen contained in the rapid release granules in the sustained release formulation may be from 1:1 to 100:1, but not limited thereto.

The water-insoluble polymer constituting the granules may be selected from the group consisting of water-insoluble cellulose or derivatives thereof, polymethacrylate, and a mixture of two or more species of polymethacrylate and polyalkylacrylate, but not limited thereto. Among these water-insoluble polymers, ethylcellulose is preferred, and more preferably, an ethylcellulose having a viscosity of 7 to 14 mPa·s can be used.

For the sustained release base material used for the coating of the granules, a water-insoluble polymer can be used, and in particular, ethylcellulose is preferred.

The sustained release pellets comprising the granules coated with the sustained release base material, preferably have a diameter of 0.05 to 2 mm.

In another aspect, the present invention provides a method for preparing a multiple unit type sustained release formulation, and in particular, the invention provides a method for preparing a multiple unit type sustained release tablet, comprising the steps of:
preparing granules containing zaltoprofen and a water-insoluble polymer;
coating the granules with a sustained release base material to prepare sustained release pellets;
preparing rapid release granules containing zaltoprofen, and
mixing the sustained release pellets and the rapid release granules with pharmaceutically acceptable additives, and tabletting the mixture.

The invention also provides a method for preparing a multiple unit type sustained release capsule, comprising the steps of:
preparing granules containing zaltoprofen and a water-insoluble polymer;
coating the granules with a sustained release base material to prepare sustained release pellets;
preparing rapid release granules containing zaltoprofen, and
filling a hard capsule with the sustained release pellets and the rapid release granules.

### Brief Description of the Drawings

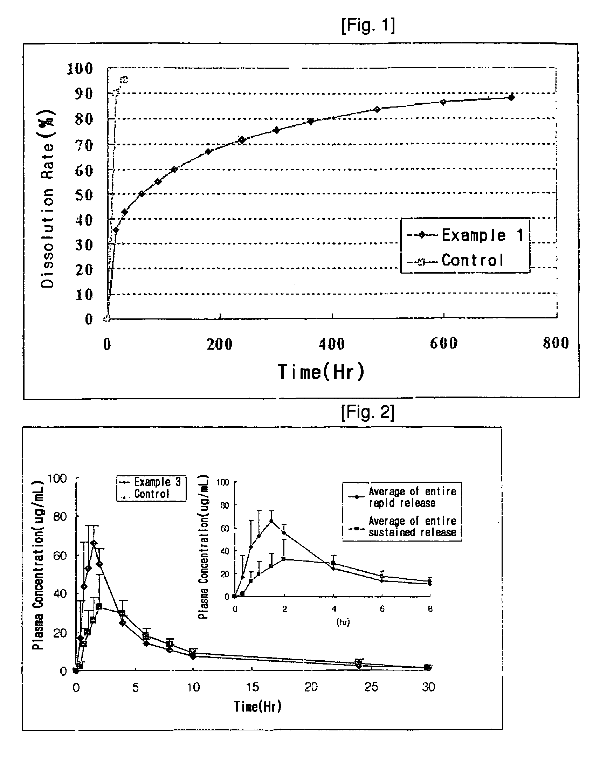

Fig. 1 is a graph showing the results of a drug dissolution test performed with multiple unit type sustained release coated tablets containing 240 mg of zaltoprofen prepared according to Example 3 of the present invention, in comparison with commercially available Soleton tablets (CJ Corp., ROK) containing 80 mg of zaltoprofen as a control.
Fig. 2 is a graph showing the average drug plasma concentration profile with time, obtained by administering a daily dose of 240 mg once to a beagle dog using the multiple unit type sustained release coated tablets containing 240 mg of zaltoprofen prepared according to Example 3 of the invention, in comparison with the commercially available Soleton tablets (CJ Corp., S. Korea) containing 80 mg of zaltoprofen as a control.

### Best Mode for Carrying Out the Invention

Hereinafter, the present invention will be described in more detail.

The invention relates to a multiple unit type sustained release. The inventors of the present invention conducted research on formulations for drugs which require rapid release of the active ingredient for rapid manifestation of the efficacy and also continuous release of for a dosage of once daily for zaltoprofen, a non-steroidal anti-inflammatory drug, and as a result, found that a formulation which facilitates rapid initial drug release as well as continuous control of drug release can be obtained by introducing rapid release granules containing zaltoprofen for rapid drug release, and introducing sustained release pellets formed from granules containing a water-insoluble polymer and zaltoprofen, the granules being coated with a sustained release base material for continuous drug release, into a single formulation, thus completing the invention.

Therefore, the sustained release formulation provided by the invention is a multiple unit type sustained release oral formulation comprising:
sustained release pellets formed of granules containing zaltoprofen and a water-insoluble polymer, the granules being coated with a sustained release base material; and
rapid release granules containing zaltoprofen. Such sustained release oral formulation may be specifically in the form of a tablet or a capsule, but the invention is not limited thereto.

The granules constituting the sustained release pellets contain zaltoprofen and a water-insoluble polymer, and are prepared by a conventional method for preparing granules, into a form in which zaltoprofen is homogeneously dispersed in the water-insoluble polymer. The water-insoluble polymer constituting these granules can be used in controlling the pattern for sustained release of zaltoprofen, by controlling the type of the water-insoluble polymer and the mixing ratio of the water-insoluble polymer and zaltoprofen, with the solubility of zaltoprofen in water being taken into consideration.

The mixing ratio of zaltoprofen and the water-insoluble polymer, which form the granules, may be appropriately selected from a range which enables control of the dissolution of zaltoprofen, but preferably, the water-insoluble polymer may be present in an amount ranging from 5 to 30 parts by weight, more preferably from 10 to 20 parts by weight, relative to 100 parts by weight of zaltoprofen in the sustained release pellet.

The water-insoluble polymer constituting such granules may be any water-insoluble polymer that is known to be appropriate for the use in the field of pharmaceutics, and specifically can be selected from the group consisting of water-insoluble cellulose or derivatives thereof, polymethacrylate, and a mixture of two or more species of polymethacrylate and polyalkyl acrylate. The water-insoluble cellulose or a derivative thereof may be exemplified by cellulose acetate, cellulose acetate phthalate, hydroxypropylene methylcellulose phthalate, ethylcellulose, or the like. The mixture of two or more species of polymethacrylate and polyalkyl acrylate may be exemplified by a mixture of polymethacrylate and polymethyl methacrylate at a ratio of 1:1, or a mixture comprising polyethyl acrylate, polymethyl methacrylate and polytrimethylam-monioethyl methacrylate chloride at a ratio of either 1:2:0.1 or 1:2:0.2. These water-insoluble polymers may be used individually or in combination. For the water-insoluble polymer, ethylcellulose may be preferably used, and an ethylcellulose having a viscosity of 7 to 14 mPa·s may be most preferably used.

When the granules are coated with a sustained release base material to form sustained release pellets, the release of zaltoprofen can be further controlled. When such coating of sustained release base material is additionally introduced, the control of the release of zaltoprofenmay be further facilitated, and the release of zaltoprofen contained in the sustained release pellets can be controlled over a prolonged time period.

The amount of the sustained release base material used in coating the granules may be appropriately selected from a range which enables control of the dissolution of zaltoprofen, but the sustained release base material can be used in an amount ranging from 5 to 40 parts by weight, preferably from 10 to 20 parts by weight, relative to 100 parts by weight of zaltoprofen in the sustained release pellets. The water-insoluble polymers mentioned above can be used as the sustained release base material used in preparing the pellets, and ethylcellulose can be preferably used.

The sustained release pellets formed by coating the granules with the sustained release base material, preferably have a particle size ranging from 0.05 to 2 mm, which size is appropriate for formulating into the form of oral formulation, particularly in the form of a tablet or a capsule.

The rapid release granules containing zaltoprofen, which constitute another part of the multiple unit type sustained release formulation according to the invention, are intended to rapidly release a portion of the drug upon administration of the sustained release formulation, thus to minimize the time taken by the drug to reach the effective blood concentration. The rapid release granules can be prepared by a conventional method known in the art for preparing rapid release granules.

the non-steroidal anti-inflammatory drug zaltoprofen

The sustained release formulation of the present invention contains the same active ingredient in the rapid release granules as well as in the sustained release pellets, so that the sustained release formulation allows, as the main purpose, rapid release as well as continuous release of the drug, in order to maintain the activity of the drug both rapid and continuous at the effective blood concentration of the drug even through administration of the drug once a day.

The multiple unit type sustained release formulation can be prepared specifically into a sustained release formulation in the form of a tablet or a capsule.

The multiple unit type sustained release tablet can be prepared by a method comprising:
preparing granules containing zaltoprofen and a water-insoluble polymer;
coating the granules with a sustained release base material to prepare sustained release pellets;
preparing rapid release granules containing zaltoprofen, and
mixing the sustained release pellets and the rapid release granules with pharmaceutically acceptable additives, and tabletting the mixture.

In the preparation of the granules, first the water-insoluble polymer by itself is dissolved in an organic solvent or dispersed in distilled water to prepare a solution or dispersion of the water-insoluble polymer, and then granules are prepared from the solution or dispersion by a conventional process for preparing granules. For the process for preparing granules, for example, a wet granulation process or a dry granulation process may be used. For the wet granulation process, a method using a fluidized bed granulator or a method using a high speed mixer can be used, while for the dry granulation method, a method for ribbon-type granulation using a roller compactor, a method for direct tabletting using a water-insoluble polymer raw material which is an excipient for direct tabletting, or the like can be used. In particular, in the case of using a fluidized bed granulator, the fluidized bed granulator is sufficiently dried and pre-heated under the conditions of an inlet temperature in the range of 60 to 85°C, and an outlet temperature of 30 to 65°C, and then granules can be prepared by adsorbing the solution of water-insoluble polymer on zaltoprofen at a rate of 300 mL/hour to 1500 mL/hour. The most suitable spraying conditions include an input temperature of 65 to 75°C, an exhaust temperature of 30 to 45°C, and an input amount of the mixture solution of 720 mL/hour.

In order to apply the sustained release base material to the granules containing zaltoprofen and the water-insoluble polymer, a solution of a water-insoluble polymer can be produced as the sustained release base material, and can be applied as a sustained release coating according to a conventional method for coating granules. The solution of the water-insoluble polymer which is used as such a sustained release base material, also can be used by dissolving a water-insoluble polymer alone in an organic solvent or in distilled water in the same manner as in the preparation of the solution or dispersion of the water-insoluble polymer used in the preparation of the granules, or by dissolving or dispersing the water-insoluble polymer together with an organic acid in an organic solvent or in distilled water. An aqueous solution of such sustained release base material may further contain one species of lubricant selected from talc, titanium oxide, light anhydrous silicic acid, and the like, while the aqueous dispersion may further contain a pharmaceutically acceptable additive such as a plasticizer, such as polyethylene glycol or triacetine. For the granule coating process, specifically a method using a fluidized bed granulator may be used. Here, the sustained releasability of the drug may be further controlled by the coating thickness of the sustained release base material thus formed.

The rapid release granules containing zaltoprofen can be prepared by a granulation method selected from a method of using a fluidized granulator according to a wet granulation process, with additives which are pharmaceutically acceptable excipient, such as a binder and a disintegrant, which are conventionally used; and a method of using a high speed mixer.

The sustained release tablets according to the invention can be prepared by mixing the sustained release pellets and the rapid release granules at a predetermined ratio, adding at least one pharmaceutically additive selected from an excipient, a lubricant, a colorant and the like, which are conventionally used for the production of tablets, and tabletting the mixture. In such a sustained release formulation, the ratio of zaltoprofen contained in the sustained release pellets to zaltoprofen contained in the rapi release granules may be adjusted to the range of 1:1 to 100:1, preferably in the range of 7:3 to 9:1.

The excipient may be preferably selected from the group consisting of lactose, microcrystalline cellulose, corn starch, potato starch, wheat starch, sucrose, D-mannitol, precipitated calcium carbonate, dextrin, pre-gelatinized starch, and combinations thereof. The excipient may be contained in an amount of 10 to 90 parts by weight based on the total weight of the tablet.

The binder may be preferably selected from the group consisting of polyvinylpyrrolidone, hydroxypropylcellulose, direct tabletted microcrystalline cellulose, hydroxypropylmethylcellulose, dextrin, gelatin, methylcellulose, hydroxyethylcellulose, hydroxymethylcellulose, polyvinyl alcohol, paste, arabic gum, and combinations thereof, and may be used in an amount of 2 to 40 parts by weight based on the total weight of the tablet.

The disintegrant may be preferably selected from the group consisting of sodium starch glycolate, crosspovidone, cross carmellose sodium, low-substituted hydroxypropylcellulose, starch, carboxymethylcellulose calcium, and combinations thereof, and the disintegrant may be contained in an amount of 0.1 to 32 parts by weight based on the total weight of the tablet composition.

The lubricant may be preferably selected from the group consisting of magnesium stearate, talc, light anhydrous silicic acid, and combinations thereof, and the lubricant may be contained in an amount of 0.1 to 20 parts by weight based on the total weight of the tablet.

For the colorant, at least one species which can be selected from titanium dioxide, iron oxide, magnesium carbonate, calcium sulfate, magnesium oxide, magnesium hydroxide, aluminum lakes, for example, Blue No. 1 Aluminum Lake, Red No. 40 Aluminum Lake, and the like can be contained in the tablet.

The sustained release tablet thus prepared can be further subjected to a process of film coating. For the film coating agent, an enteric or non-enteric film coating agent may be used, and the enteric film coating agent can be cellulose acetate phthalate (CAP), polyvinyl acetate phthalate (PVAP), a methacrylate polymer (Eudragit L, S), or the like, while the non-enteric film coating agent can be hydroxypropylcellulose (HPC), methylcellulose (MC), ethylcellulose (EC), hydroxypropylmethylcellulose (HPMC), povidone (PVP), polyvinyl alcohol (PVA), cellulose acetate (CA), shellac, or the like. The process of such tablet coating can be performed by, for example, a pan coating method, a fluidized bed coating method, a compression coating method, or the like.

Among the multiple unit type sustained release oral formulations described above, the capsule can be prepared by mixing the sustained release pellets and the rapid release granules prepared in the same manner as in the preparation of tablets, and filling a hard capsule with the resulting mixture. The filling of the capsule can be performed by a conventional method such as fluidizing the pellets under pressure to fill a hard capsule, filling a hard capsule by means of free-fall, or the like.

The amount of the sustained release formulation according to the invention to be administered to a human body may be appropriately selected in accordance with the absorption rate in the body, rate of inactivation, rate of excretion, the age, gender and condition of the patient, severity of the disease, or the like.

### Mode for the Invention

Hereinafter, the present invention will be described in more detail with reference to Examples. However, these Examples are for the illustrative purpose only, and the invention is not intended to be limited by these Examples.

### EXAMPLE 1

### A. Preparation of water-insoluble polymer solution

100 g of an ethylcellulose having a viscosity of 14 mPa·s, which is a water-insoluble polymer, was added to 1000 g of a 80% aqueous ethanol solution, and then the mixture was stirred at 1000 rpm for 30 to 60 minutes using a mechanical mixer to prepare a solution of the water-insoluble polymer.

### B. Preparation of granules comprising zaltoprofen and water-insoluble polymer

A fluidized bed granulator was sufficiently dried and pre-heated under the conditions of an inlet temperature of 65°C and an outlet temperature of 30°C, and then the solution of water-insoluble polymer ethylcellulose thus produced was adsorbed onto 500 g of zaltoprofen at an input rate of 720 mL/hour to produce 600 g of granules.

### C. Preparation of sustained release base material coating solution

75 g of an ethylcellulose having a viscosity of 14 mPa·s, which is a sustained release base material, was added to 750 g of a 80% aqueous ethanol solution, and then the mixture was stirred at 1000 rpm for 30 to 60 minutes using a mechanical mixer. Then, 15 g of talc was added and mixed thereto.

### D. Preparation of sustained release pellets

600 g of the granules prepared in the step B above were sprayed using a fluidized granulator, and the sustained release material coating solution prepared in the step C above was sprayed at an input rate of 720 mL/hour to produce 690 g of sustained release pellets having a diameter in the range of 0.05 to 1.5 mm.

### E. Preparation of rapid release granules

12 g of a polyvinylpyrrolidone binder having a molecular weight of 30,000 to 50,000 kg/mol was mixed with 60 g of a 50% aqueous ethanol solution, and the binder was dissolved therein while stirring at a rate of 600 rpm using a mechanical mixer, to prepare a binding solution.

72 g of zaltoprofen, 90 g of microcrystalline cellulose, and 60 g of sodium starch gluconate were mixed, and then rapid release granules of zaltoprofen were prepared while introducing the previously prepared binding solution into a high speed mixer. The operating conditions for the high speed mixer were as shown in Table 1 below.

**Table 1**

| | Chopper(rpm) | Stirrer (rpm) | Time (min) |
|---|---|---|---|
| Compounding | 1000 | 150 | 10 |
| Mixing | 1500 | 200 | 15 |
| Granulating | 1200 | 200 | 15 |

After preparing the granules under the above-described conditions, the granules were dried until the weight loss on drying at 40°C became 3% or less, and then screened using an oscillator with a 25-35 mesh size.

### F. Preparation of multiple unit type sustained release tablets

Sustained release tablets were produced by a direct tabletting method. 298.08 g of the sustained release pellets of zaltoprofen as prepared in the above were mixed with 78 g of the rapid release granules prepared in the step E, and 2.92 g of magnesium stearate, and then the mixture was formed into tablets of 379 mg having a hardness of 7 to 12 Kp.

### EXAMPLE 2: Preparation of multiple unit type sustained release capsules

298.08 g of the sustained release pellets of zaltoprofen prepared in the steps D and E of Example 1, 78 g of the rapid release granules, and 2.92 g of magnesium stearate were together filled in hard capsules No. 0 of 379 mg by a free falling method, to produce sustained release capsules.

### EXAMPLE 3: Preparation of multiple unit type sustained release coated tablets

### A. Preparation of multiple unit type sustained release tablets

Sustained release tablets were prepared by the same method as in Example 1.

### B. Film coating of sustained release tablets

20 g of a coating agent, Opadry® AMB (PVA; Colorcon, Inc.) was suspended in 200 g of distilled water to prepare a coating suspension, and the sustained release tablets of zaltoprofen prepared in the step A were filled in a coating pan (Hi-coater). The dried tablets in the coating pan was maintained under the conditions of a suction air temperature of 75 to 85°C and an exhaust air temperature of about 35 to 45°C. The coating suspension was sprayed onto the dried tablets using a pneumatically operated spraying apparatus, and then air supply was continued for another 30 to 40 minutes to dry the coated tablets. The amount of coating of the Opadry (PVA; Colorcon, Inc.) coating material on the tablets thus obtained was 2.11 % based on the total weight of the tablet.

The prescriptions of the formulations prepared in Examples 1 to 3 are as shown in Table 2 below.

**Table 2**

| | Component | Example 1 | Example 2 | Example 3 |
|---|---|---|---|---|
| | | (mg) | | |
| Active ingredient | Zaltoprofen | 216 | 216 | 216 |
| Granulating agent | Ethylcellulose, 14 mPa·s | 43.2 | 43.2 | 43.2 |
| Granule coating agent | Ethylcellulose, 14 mPa·s | 32.4 | 32.4 | 32.4 |
| Lubricant | Talc | 6.48 | 6.48 | 6.48 |
| | Sustained Granules | 298.08 | 298.08 | 298.08 |
| Active ingredient | Zaltoprofen | 24 | 24 | 24 |
| Excipient | Microcrystalline cellulose | 30 | 30 | 30 |
| Disintegrant | Sodium starch gluconate | 20 | 20 | 20 |
| Binder | PVP K30 | 4 | 4 | 4 |
| | Rapid release granules | 78 | 78 | 78 |
| Lubricant | Mg-stearate | 2.92 | 2.92 | 2.92 |
| | Sustained or hard capsules | 379.00 | 379.00 | 379.00 |
| Film coating base material | Opadry® AMB | | | 8.00 |
| | Film coated tablets | | | 387 |

### EXPERIMENTAL EXAMPLE: Dissolution test and absorption test

### A. In vitro assay

A dissolution test was carried out on the multiple unit type sustained release tablets prepared in Example 3 above. The procedure of the dissolution test followed the Dissolution Test No. 2 among the Korean Pharmacopeia General Test Methods, and the test was performed on the tablets and capsules prepared in the above, using water or a pH 7.8 buffer solution as an eluent at a rate of 100 rpm/min for 12 hours. The eluent was taken in an amount of 5 ml each at 15 min, 30 min, 60 min, 90 min, 2 hours, 3 hours, 4 hours, 5 hours, 6 hours, 8 hours, 10 hours and 12 hours after the initiation of the dissolution test, and was filtered. The filtrate was used as the test liquid in an analysis by high performance liquid chromatography. A commercially available product Soleton (rapid release formulation) was used as the control. The results are presented in Fig. 1.

Referring to Fig. 1, the dissolution rate of the sustained release formulation of Example 1 was shown to be 20 to 40% in 1 hour, 40 to 60% in 3 hours, and 80% or more in 12 hours. From these results, it can be seen that the sustained release formulation of the present invention is capable of initially releasing a relatively large amount of drug as well as continuously releasing drug over 12 hours, thus the formulation being a formulation which exhibits the effects immediately upon administration and requires to be administered only once a day. Such release pattern was distinctive from the release pattern of the control used, the commercially available rapid release formulation Soleton® which showed 100% dissolution in a short time.

### B. In vivo assay

Beagle dogs (Marshall Beijing, China, 13 months old, average weight 11.5 kg, male) were fasted for a whole day before the administration of the test material, and the sustained release tablets of zaltoprofen prepared in Example 3 above and commercially available Soleton tablets were orally administered by force. After the administration, 10 mL of water was forcibly administered. The dosage regimen was as indicated in Table 3.

**Table 3**

| Administration Group | Material administered | Gender | No. of animals | Animal Reference number | Dose (mg/animal) |
|---|---|---|---|---|---|
| T1 | 1^{st} -Example 32 ^{nd} -Soleton | Male | 3 | A-CD-F | 240 mg tablet/animal |
| T2 | 1^{st} -Soleton2^{nd} -Example 3 | Male | 3 | D - FA - C | 240 mg tablet/animal |

Blood samples were taken from the test animals, and sampling was conducted at 20 min, 40 min, 1 hour, 1.5 hours, 2 hours, 4 hour, 6 hours, 8 hours, 10 hours, 24 hours and 30 hours after administration of the drug. 1 ml of blood was taken from the cephalic vein or jugular vein according to the sampling schedule depending on the group constitution. The sampled blood was contained in a container which had been treated with an anticoagulant (EDTA) or heparin, and centrifuged at 13,000 rpm for 3 minutes to separate and analyze the plasma. When the separated plasma was not subjected to analysis immediately, the plasma was kept in a freezer (-20°C) until the time of analysis.

The samples were pre-treated by adding 100 □ of an internal standard solution of diphenyloxazole (25 □/ml) to 200 □ of the plasma, then adding 100 □ of 2 M acetic acid and 1 ml of dichloromethane thereto, and shaking the mixture for 40 seconds. The drug was extracted from the plasma, and was analyzed by HPLC to give an average blood concentration profile as shown in Fig. 2. Furthermore, the average blood concentration of the entire sustained pellets and the average blood concentration of the entire rapid release granules of the sustained release formulation of Example 3 were determined, and the results are shown in Fig. 2.

Based on these blood concentration profiles, the general in vivo pharmacokinetic parameters such as the maximum blood concentration (Cₘₐₓ), time to attain the maximum blood concentration (Tₘₐₓ), the area under the concentration curve (AUC) and the like were calculated according to a non-compartment model analysis, and the results are presented in Table 4.

**Table 4**

| Comparison of pharmacokinetic parameters of zaltoprofen sustained release tablets and Soleton® tablets | | |
|---|---|---|
| | Example 3 | Soleton® tablets |
| Cₘₐₓ (mg/ml) | 37.79 ±12.27 | 66.99 ± 9.85 |
| Tₘₐₓ (hr) | 2.17 ±0.98 | 1.28 ± 0.36 |
| AUC₀₋ₜ (ug hr/ml) | 98.97 ±73.77 | 26.61 ± 65.64 |
| RBA | - | 92% |

From the results of the blood concentration over time in Fig. 2 and the pharmacokinetic parameters in Table 4, it can be seen that the sustained release tablets of zaltoprofen prepared in Example 2 were more effectively used in the body compared with the control Soleton tablets.

### Industrial Applicability

As discussed above, according to the present invention, there can be provided a sustained release formulation which exhibits sustained release to the extent that administration of once a day is made possible for the convenience of dosage and patient compliance, as well as exhibits rapid release immediately after administration so that the effective blood concentration of drug can be attained rapidly. This sustained release formulation can be usefully applied to the cases where sustained release is required due to the nature of the drug, as well as rapid manifestation of efficacy is required.

## Claims

1. A multiple unit type sustained release oral formulation comprising:
sustained release pellets formed from granules containing zaltoprofen and a water-insoluble polymer, the granules being coated with a sustained release base material; and rapid release granules containing zaltoprofen.

2. The multiple unit type sustained release oral formulation according to claim 1, which is **characterized by** being in the form of a tablet or a capsule.

3. The multiple unit type sustained release oral formulation according to claim 1, wherein the granules are **characterized by** containing zaltoprofen and the water-insoluble polymer contain 5 to 30 parts by weight of the water-insoluble polymer, relative to 100 parts by weight of zaltoprofen in the sustained release pellets.

4. The multiple unit type sustained release oral formulation according to claim 1, wherein the sustained release pellets are **characterized by** being coated with 5 to 40 parts by weight of the sustained release base material relative to 100 parts by weight of zaltoprofen in the sustained release pellets.

5. The multiple unit type sustained release oral formulation according to claim 1, wherein the ratio of zaltoprofen contained in the sustained release pellets to zaltoprofen contained in the rapid release granules is **characterized by** being from 1:1 to 100:1.

6. The multiple unit type sustained release oral formulation according to claim 1, wherein the water-insoluble polymer is **characterized by** benig selected from the group consisting of water-insoluble cellulose or derivatives thereof, polymethacrylate, and a mixture of two or more species of polymethacrylate and polyalkyl acrylate.

7. The multiple unit type sustained release oral formulation according to claim 6, wherein the water-insoluble polymer is **characterized by** being an ethylcellulose having a viscosity of 7 to 14 mPa·s.

8. The multiple unit type sustained release oral formulation according to claim 1, wherein the sustained release base material is **characterized by** being a water-insoluble polymer.

9. The multiple unit type sustained release oral formulation according to claim 8, wherein the water-insoluble polymer is ethylcellulose.

10. The multiple unit type sustained release oral formulation according to claim 1, wherein the sustained release pellets are **characterized by** having a diameter of 0.05 to 2 mm.

11. A method for preparing a multiple unit type sustained release tablet, comprising the steps of:
preparing granules containing zaltoprofen and a water-insoluble polymer;
coating the granules with a sustained release base material to prepare sustained release pellets;
preparing rapid release granules containing zaltoprofen; and
mixing the sustained release pellets and rapid release granules together with pharmaceutically acceptable additives, and tabletting the mixture.

12. A method for preparing a multiple unit type sustained release capsule, comprising the steps of:
preparing granules containing zaltoprofen and a water-insoluble polymer;
coating the granules with a sustained release base material to prepare sustained release pellets;
preparing rapid release granules containing zaltoprofen; and
filling the sustained release pellets and rapid release granules into a hard capsule.

## Patentansprüche

1. Aus mehreren Einheiten bestehende orale Formulierung mit verzögerter Freisetzung, welche das Folgende umfasst:
Pellets mit verzögerter Freisetzung, welche aus Kügelchen gebildet sind, die Zaltoprofen und ein wasserunlösliches Polymer enthalten, wobei die Kügelchen mit einem Basismaterial für verzögerte Freisetzung beschichtet sind; und Kügelchen mit schneller Freisetzung, welche Zaltoprofen enthalten.

2. Aus mehreren Einheiten bestehende orale Formulierung mit verzögerter Freisetzung nach Anspruch 1, **dadurch gekennzeichnet, dass** sie in Form einer Tablette oder Kapsel vorliegt.

3. Aus mehreren Einheiten bestehende orale Formulierung mit verzögerter Freisetzung nach Anspruch 1, wobei die Kügelchen, die **dadurch gekennzeichnet sind, dass** sie Zaltoprofen und das wasserunlösliche Polymer enthalten, 5 bis 30 Gewichtsteile des wasserunlöslichen Polymers in Bezug auf 100 Gewichtsteile des Zaltoprofens in den Pellets mit verzögerter Freisetzung enthalten.

4. Aus mehreren Einheiten bestehende orale Formulierung mit verzögerter Freisetzung nach Anspruch 1, wobei die Pellets mit verzögerter Freisetzung **dadurch gekennzeichnet sind, dass** sie mit 5 bis 40 Gewichtsteilen des Basismaterials für verzögerte Freisetzung beschichtet sind, bezogen auf 100 Gewichtsteile Zaltoprofen in den Pellets mit verzögerter Freisetzung.

5. Aus mehreren Einheiten bestehende orale Formulierung mit verzögerter Freisetzung nach Anspruch 1, wobei das Verhältnis des Zaltoprofens, das in den Pellets mit verzögerter Freisetzung enthalten ist, zu dem Zaltoprofen, das in den Kügelchen mit schneller Freisetzung enthalten ist, **dadurch gekennzeichnet ist, dass** es von 1:1 bis zu 100:1 beträgt.

6. Aus mehreren Einheiten bestehende orale Formulierung mit verzögerter Freisetzung nach Anspruch 1, wobei das wasserunlösliche Polymer **dadurch gekennzeichnet ist, dass** es aus der Gruppe ausgewählt ist, die aus besteht, die aus wasserunlöslicher Cellulose oder Derivaten dieser, Polymethacrylat und einem Gemisch aus zwei oder mehr Teilchen Polymethacrylat und Polyalkylacrylat besteht.

7. Aus mehreren Einheiten bestehende orale Formulierung mit verzögerter Freisetzung nach Anspruch 6, wobei das wasserunlösliche Polymer **dadurch gekennzeichnet ist, dass** es sich um eine Ethylcellulose einer Viskosität von 7 bis 14 mPa·s handelt.

8. Aus mehreren Einheiten bestehende orale Formulierung mit verzögerter Freisetzung nach Anspruch 1, wobei das Basismaterial für verzögerte Freisetzung **dadurch gekennzeichnet ist, dass** es sich um ein wasserunlösliches Polymer handelt.

9. Aus mehreren Einheiten bestehende orale Formulierung mit verzögerter Freisetzung nach Anspruch 8, wobei es sich bei dem wasserunlöslichen Polymer um Ethylcellulose handelt.

10. Aus mehreren Einheiten bestehende orale Formulierung mit verzögerter Freisetzung nach Anspruch 1, wobei die Pellets mit verzögerter Freisetzung **dadurch gekennzeichnet sind, dass** sie einen Durchmesser von 0,05 bis 2 mm aufweisen.

11. Verfahren zur Herstellung einer aus mehreren Einheiten bestehenden Tablette mit verzögerter Freisetzung, welches die folgenden Schritte umfasst:
Herstellen von Kügelchen, welche Zaltoprofen und ein wasserunlösliches Polymer enthalten;
Beschichten der Kügelchen mit einem Basismaterial für verzögerte Freisetzung, um Pellets mit verzögerter Freisetzung herzustellen;
Herstellen von Kügelchen mit schneller Freisetzung, welche Zaltoprofen enthalten; und
Vermischen der Pellets mit verzögerter Freisetzung und Kügelchen mit verzögerter Freisetzung zusammen mit pharmazeutisch unbedenklichen Additiven und Tablettieren des Gemisches.

12. Verfahren zur Herstellung einer aus mehreren Einheiten bestehenden Kapsel mit verzögerter Freisetzung, welches die folgenden Schritte umfasst:
Herstellen von Kügelchen, welche Zaltoprofen und ein wasserunlösliches Polymer enthalten;
Beschichten der Kügelchen mit einem Basismaterial für verzögerte Freisetzung, um Pellets mit verzögerter Freisetzung herzustellen;
Herstellen von Kügelchen mit schneller Freisetzung, welche Zaltoprofen enthalten; und
Füllen der Pellets mit verzögerter Freisetzung und der Kügelchen mit verzögerter Freisetzung in eine Hartkapsel.

## Revendications

1. Formulation pour voie orale à libération prolongée et à plusieurs unités comprenant: des pastilles à libération prolongée formées de granules comprenant zaltoprofen et un polymère insoluble à l'eau, les granules étant revêtus avec un matériau de base à libération prolongée; et des granules à libération rapide comprenant zaltoprofen.

2. Formulation pour voie orale à libération prolongée et à plusieurs unités selon la revendication 1, qui est **caractérisée par** étant dans la forme d'une tablette ou une capsule.

3. Formulation pour voie orale à libération prolongée et à plusieurs unités selon la revendication 1, où les granules sont **caractérisés par** comprenant zaltoprofen et un polymère insoluble à l'eau contenant 5 à 30 parties en poids du polymère insoluble à l'eau, relatif à 100 parties en poids de zaltoprofen dans les pastilles à libération prolongée.

4. Formulation pour voie orale à libération prolongée et à plusieurs unités selon la revendication 1, où les pastilles à libération prolongée sont **caractérisées par** étant revêtues avec 5 à 40 parties en poids du matériau de base à libération prolongée relatif à 100 parties en poids de zaltoprofen dans les pastilles à libération prolongée.

5. Formulation pour voie orale à libération prolongée et à plusieurs unités selon la revendication 1, où le rapport du zaltoprofen contenu dans les pastilles à libération prolongée au zaltoprofen contenu dans les granules à libération rapide est **caractérisé par** étant de 1:1 à 100:1.

6. Formulation pour voie orale à libération prolongée et à plusieurs unités selon la revendication 1, où le polymère insoluble à l'eau est **caractérisé par** étant sélecté du groupe formé de cellulose insoluble à l'eau ou de dérivés de celle-ci, de polyméthacrylate, et une mixture de deux ou plusieurs espèces de polyméthacrylate et d'acrylate de polyalkyle.

7. Formulation pour voie orale à libération prolongée et à plusieurs unités selon la revendication 6, où le polymère insoluble à l'eau est **caractérisé par** étant un éthylcellulose ayant une viscosité de 7 à 14 mPa·s.

8. Formulation pour voie orale à libération prolongée et à plusieurs unités selon la revendication 1, où le matériau de base à libération prolongée est **caractérisé par** étant un polymère insoluble à l'eau.

9. Formulation pour voie orale à libération prolongée et à plusieurs unités selon la revendication 8, où le polymère insoluble à l'eau est l'éthylcellulose.

10. Formulation pour voie orale à libération prolongée et à plusieurs unités selon la revendication 1, où les pastilles à libération prolongée sont **caractérisées par** ayant un diamètre de 0,05 à 2 mm.

11. Procédé pour préparer une tablette à libération prolongée et à plusieurs unités comprenant les étapes de:
- préparer les granules contenant zaltoprofen et un polymère insoluble à l'eau;
- revêtir les granules avec un matériau de base à libération prolongée pour préparer les pastilles à libération prolongée;
- préparer les granules à libération rapide contenant zaltoprofen; et
- mélanger les pastilles à libération prolongée et les granules à libération rapide ensemble avec des additifs acceptables du point de vue pharmaceutique et faire des tablettes de la mixture.

12. Procédé pour préparer une capsule à libération prolongée et à plusieurs unités comprenant les étapes de:
- préparer les granules contenant zaltoprofen et un polymère insoluble à l'eau;
- revêtir les granules avec un matériau de base à libération prolongée pour préparer les pastilles à libération prolongée;
- préparer les granules à libération rapide contenant zaltoprofen; et
- remplir les pastilles à libération prolongée et les granules à libération rapide dans une capsule dure.
